# EUROPÄISCHE PATENTSCHRIFT

(11) **EP 3 422 671 B1**
(45) Veröffentlichungstag und Bekanntmachung des Hinweises auf die Patenterteilung: **29.07.2020**
(21) Anmeldenummer: 17178387.1
(22) Anmeldetag: 28.06.2017
(51) Int. Cl.: H04L 29/08, H04W 4/00

(54) **ERFASSUNGSVORRICHTUNG FÜR EINE AKTIVE BLENDSCHUTZVORRICHTUNG**
CAPTURE DEVICE FOR AN ACTIVE GLARE SHIELD DEVICE
DISPOSITIF DE DÉTECTION POUR UN DISPOSITIF ANTI-ÉBLOUISSEMENT ACTIF

(43) Veröffentlichungstag der Anmeldung: 02.01.2019
(73) Patentinhaber: Optrel Holding AG, 9630 Wattwil (CH)
(72) Erfinder: Hofer Kraner, Ramon, 9100 Herisau (CH); Pittorino, Tindaro, 9470 Buchs/SG (CH); Schreiber, Olaf, 9470 Buchs/SG (CH)
(74) Vertreter: Daub, Thomas

(56) Entgegenhaltungen:
- WO-A1-02/49554
- WO-A1-2015/200492
- US-A1- 2007 221 636
- US-A1- 2014 320 771
- Anonymous: "Block code - Wikipedia", , 20. Juni 2017 (2017-06-20), XP055421459, Gefunden im Internet: URL:https://en.wikipedia.org/w/index.php?t itle=Block_code&oldid=786615062 [gefunden am 2017-11-02]

## Beschreibung

### Stand der Technik

Die Erfindung betrifft eine Erfassungsvorrichtung für eine aktive Blendschutzvorrichtung. Es ist bereits eine Erfassungsvorrichtung für eine aktive Blendschutzvorrichtung, mit einer Erfassungseinheit, welche zu einer direkten oder indirekten Erfassung zumindest eines Schweißparameters eines Schweißgeräts vorgesehen ist, und mit zumindest einer Kommunikationseinheit, welche zu einer Übertragung zumindest einer Information des zumindest einen Schweißparameters an die aktive Blendschutzvorrichtung vorgesehen ist, vorgeschlagen worden.

Die Aufgabe der Erfindung besteht insbesondere darin, eine gattungsgemäße Vorrichtung mit verbesserten Eigenschaften hinsichtlich einer Übertragungsgeschwindigkeit sowie einer Übertragungssicherheit, insbesondere der Kommunikationseinheit, bereitzustellen. Die Aufgabe wird erfindungsgemäß durch die Merkmale des Patentanspruchs 1 gelöst während vorteilhafte Ausgestaltungen und Weiterbildungen der Erfindung den Unteransprüchen entnommen werden können.

WO 02/49554 A1 beschreibt eine Blendschutzvorrichtung, welche unempfindlicher ist gegen Einflüsse von Fremdlicht und welche eine einfachere, bessere und sicherere Steuerung der Schutzfilter ohne Fehlverhalten ermöglicht.

US 2007/221636 A1 beschreibt ebenfalls eine verbesserte Blendschutzvorrichtung.

### Vorteile der Erfindung

Die Erfindung gemäß Anspruch 1 geht aus von einer Erfassungsvorrichtung für eine aktive Blendschutzvorrichtung, mit einer Erfassungseinheit, welche zu einer direkten oder indirekten Erfassung zumindest eines Schweißparameters eines Schweißgeräts vorgesehen ist, und mit zumindest einer Kommunikationseinheit, welche zu einer Übertragung zumindest einer Information des zumindest einen Schweißparameters an die aktive Blendschutzvorrichtung vorgesehen ist.

Es wird vorgeschlagen, dass die Kommunikationseinheit dazu vorgesehen ist, die zumindest eine Information des zumindest einen Schweißparameters in Form einer Bitfolge definierter Länge an die aktive Blendschutzvorrichtung zu übertragen. Dadurch kann insbesondere eine vorteilhaft hohe Übertragungsgeschwindigkeit sowie eine vorteilhaft hohe Übertragungssicherheit erreicht werden. Durch die Übertragung in Form einer Bitfolge definierter Länge kann insbesondere auf zusätzliche Informationen, wie z.B. CRC und Syncword, welche bei einer paketorientierten Übertragung benötigt werden, verzichtet werden. Das Übertragungspaket kann daher wesentlich kürzer ausgeführt werden. Hierdurch kann eine vorteilhaft schnelle Datenübertragung erreicht werden. Ferner kann eine vorteilhaft hohe Robustheit des Übertragungskanals erreicht werden, da die Informationen, insbesondere Signalzustände, nicht einfach als einzelne Bits übertragen werden, sondern mittels Bitfolgen definierter Länge. Diese Darstellung ermöglicht es, eventuelle Einflüsse von Störsignalen mittels Signalverarbeitung gering zu halten. Die Übertragungszeit eines Signalzustands wird dadurch zwar entsprechend vergrößert, liegt jedoch weiterhin unterhalb einer paketorientierten Übertragung. Die Latenzzeiten werden durch die Anzahl der Bits für die Darstellung eines Signalzustands und der Modulationsrate bestimmt. Die maximale Umschaltzeit von einem zum anderen Signalzustand liegt bei der doppelten Übertragungszeit einer Bitfolge. Das resultiert aus der Unabhängigkeit zwischen dem Schaltvorgang und der Funkübertragung. Beide Vorgänge arbeiten nicht synchron. Die Zeiten für die Signalverarbeitung sind dabei nicht berücksichtigt. Die Signallaufzeiten auf der Luftschnittstelle sind bei Anwendungen mit geringen Reichweiten vernachlässigbar. Hierdurch können daher insbesondere möglichst geringe Latenzzeiten bei der Datenübertragung von den Informationen, insbesondere von zumindest zwei Signalzuständen wie z.B. zum schnellen Abdunkeln eines Schweisshelms, erreicht werden.

Unter einer "aktiven Blendschutzvorrichtung" soll in diesem Zusammenhang insbesondere eine Blendschutzvorrichtung mit einem aktiven optischen Blendschutzfilter verstanden werden. Dabei soll unter einer "Blendschutzvorrichtung" in diesem Zusammenhang insbesondere eine Vorrichtung verstanden werden, welche zu einem Schutz eines Benutzers vor zu großer Helligkeit und/oder vor Funken vorgesehen ist. Vorzugsweise soll darunter insbesondere eine Vorrichtung verstanden werden, welche zu einem Schutz von Augen und/oder einem Gesichtsbereich eines Benutzers während eines Schweiß- und/oder Schleifprozesses dient. Bevorzugt soll darunter insbesondere eine Blendschutzvorrichtung verstanden werden, welche insbesondere zu einem Schutz der Augen eines Benutzers zumindest während eines Schweißprozesses dient. Es sind verschiedene, einem Fachmann als sinnvoll erscheinende Ausgestaltungen einer Blendschutzvorrichtung denkbar, wie beispielsweise als Schweißhelm, -schirm, -maske und/oder -schild. Ferner soll dabei unter einem "aktiven optischen Blendschutzfilter" insbesondere ein optischer Filter verstanden werden, welcher insbesondere ein Schutzglas und/oder ein Kunststoffschutzglas bildet. Vorzugsweise soll darunter insbesondere ein optischer Filter verstanden werden, dessen Lichtdurchlässigkeit einstellbar ausgeführt ist. Bevorzugt soll darunter insbesondere ein optischer Schweißschutzfilter mit einer automatischen Verdunkelung verstanden werden. Besonders bevorzugt weist der Blendschutzfilter zumindest eine in der Transmission schaltbare Flüssigkristallebene auf. Es sind verschiedene, einem Fachmann als sinnvoll erscheinende Ausgestaltungen des optischen Blendschutzfilters denkbar, insbesondere soll darunter jedoch ein ADF, auch "automatic darkening filter" oder "automatischer Schweißerschutzfilter" genannt, verstanden werden. Des Weiteren soll unter einer "Erfassungseinheit" in diesem Zusammenhang insbesondere eine Einheit verstanden werden, welche zu einer direkten oder indirekten Erfassung zumindest eines Schweißparameters eines Schweißgeräts vorgesehen ist. Die Erfassungseinheit kann dabei insbesondere sowohl zu einer direkten Erfassung des Schweißparameters vorgesehen sein als auch dazu vorgesehen sein, durch Erfassung alternativer Kenngrößen auf den zumindest einen Parameter zu schließen. Vorzugsweise umfasst die Erfassungseinheit zumindest einen Sensor. Dabei soll unter einem "Sensor" in diesem Zusammenhang insbesondere ein Element verstanden werden, das dazu vorgesehen ist, zumindest eine Kenngröße und/oder eine physikalische Eigenschaft aufzunehmen, wobei die Aufnahme aktiv, wie insbesondere durch Erzeugen und Aussenden eines elektrischen Messsignals, und/oder passiv, wie insbesondere durch eine Erfassung von Eigenschaftsänderungen eines Sensorbauteils, stattfinden kann. Es sind verschiedene, einem Fachmann als sinnvoll erscheinende Sensoren denkbar. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt. Ferner soll in diesem Zusammenhang unter einem "Schweißparameter des Schweißgeräts" insbesondere ein Parameter eines Betriebs des Schweißgeräts verstanden werden. Vorzugsweise soll darunter insbesondere ein Parameter verstanden werden, welcher Rückschluss auf einen Betriebszustand, insbesondere auf einen Status, des Schweißgeräts gibt. Es sind verschiedene, einem Fachmann als sinnvoll erscheinende Schweißparameter denkbar, wie beispielsweise ein Schweißstrom.

Unter einer "Kommunikationseinheit" soll in diesem Zusammenhang insbesondere eine Einheit verstanden werden, welche zu einer Bereitstellung einer, insbesondere kabellosen, Kommunikation mit der aktiven Blendschutzvorrichtung vorgesehen ist. Vorzugsweise weist die Kommunikationseinheit zu einer Kommunikation mit der aktiven Blendschutzvorrichtung zumindest eine Schnittstelle auf. Vorzugsweise soll unter einer Kommunikationseinheit insbesondere eine Einheit verstanden werden, welche zu einem Austausch von Daten vorgesehen ist. Insbesondere weist die Kommunikationseinheit zumindest einen Informationseingang und zumindest einen Informationsausgang auf. Vorzugsweise weist die Kommunikationseinheit zumindest zwei Informationseingänge und zumindest zwei Informationsausgänge auf, wobei jeweils zumindest ein Informationseingang und zumindest ein Informationsausgang zu einer Verbindung mit einem physischen System, insbesondere dem externen Schweißgerät, vorgesehen sind. Besonders bevorzugt soll darunter eine Schnittstelle zwischen zumindest zwei physischen Systemen, wie insbesondere zwischen der aktiven Blendschutzvorrichtung und der Erfassungsvorrichtung, verstanden werden. Es sind verschiedene, einem Fachmann als sinnvoll erscheinende Kommunikationseinheiten denkbar, insbesondere soll darunter jedoch eine drahtlose Schnittstelle, wie beispielsweise Bluetooth, WLAN, Zigbee, NFC, RFID, GSM, LTE oder UMTS, und/oder eine drahtgebundene Schnittstelle, wie beispielsweise ein USB-Anschluss, eine Canbus-Schnittstelle, eine RS485 Schnittstelle, eine Ethernet-Schnittstelle, eine optische Schnittstelle, eine KNX-Schnittstelle und/oder eine Powerline-Schnittstelle, verstanden werden. Unter einer "Bitfolge definierter Länge" soll in diesem Zusammenhang insbesondere eine Folge von zumindest zwei Bits verstanden werden, wobei die Folge pro übertragener Information immer die selbe Bitanzahl aufweist. Vorzugsweise wird die Information daher immer mittels einer Folge gleichvieler Bits übertragen. Die Bitfolge weist dabei insbesondere unabhängig von der enthaltenen Information dieselbe Bitanzahl auf.

Ferner wird vorgeschlagen, dass die Kommunikationseinheit dazu vorgesehen ist, die zumindest eine Information des zumindest einen Schweißparameters in Form einer Bitfolge definierter Länge kabellos, insbesondere mittels einer Funkverbindung, an die aktive Blendschutzvorrichtung zu übertragen. Dadurch kann insbesondere eine vorteilhaft komfortable Übertragung der Informationen erreicht werden. Es kann insbesondere auf ein störendes Kabel verzichtet werden.

Des Weiteren wird vorgeschlagen, dass die Kommunikationseinheit zumindest eine Sendeeinheit, welche zu einer direkten Übertragung eines Signals an die aktive Blendschutzvorrichtung vorgesehen ist, und zumindest eine Modulationseinheit, welche dazu vorgesehen ist, den zumindest einen Schweißparameter in Form einer Bitfolge definierter Länge direkt auf das Signal zu modulieren, aufweist. Vorzugsweise ist die Sendeeinheit dazu vorgesehen ein Trägersignal zu erzeugen, auf welches die Bitfolge definierter Länge aufmoduliert werden kann. Bevorzugt wird dazu insbesondere ein proprietäres Funksignal eingesetzt. Die Kommunikation erfolgt ferner insbesondere unidirektional, wobei die Informationsübertragung über die Sendeeinheit in einem Streaming-Verfahren erfolgt. Dadurch kann insbesondere eine vorteilhaft schnelle Datenübertragung erreicht werden. Es kann insbesondere eine direkte Modulation verwendet werden, sodass auf eine zeitaufwändige Datenverarbeitung zumindest im Wesentlichen verzichtet werden kann. Es kann also insbesondere eine ungebufferte Datenübertragung, also eine direkte Modulation bzw. Demodulation ohne Einbeziehung eines Pakethandlers, verwendet werden. Unter einer "Sendeeinheit" soll in diesem Zusammenhang insbesondere eine Einheit verstanden werden, welche zu einer Erzeugung eines Signals, insbesondere eines Trägersignals, vorgesehen ist. Vorzugsweise ist die Sendeeinheit von einer Funksendeeinheit gebildet, welche zu einer Erzeugung eines Funksignals vorgesehen ist. Die Sendeeinheit bildet insbesondere eine Funkschnittstelle aus. Ferner soll in diesem Zusammenhang unter einer "Modulationseinheit" insbesondere eine Einheit erstanden verstanden werden, welche zu einer Modulation eines Signals der Sendeeinheit vorgesehen ist. Vorzugsweise soll darunter insbesondere eine Einheit verstanden werden, welche dazu vorgesehen ist, Daten und/oder Informationen in Form von Bits auf ein Signal der Sendeeinheit zu modulieren. Die Modulation kann dabei beispielsweise mittels einer Frequenzmodulation erreicht werden. Es sind verschiedene, einem Fachmann als sinnvoll erscheinende Modulationseinheiten denkbar, wie beispielsweise ein HF-Tranceiverbaustein mit direkter Modulation. Bausteine, die nur eine paketorientierte Übertragung zulassen, sind vorzugsweise nicht geeignet. Es wird jedoch keine synchrone Modulation bzw. Demodulation vorrausgesetzt.

Es wird ferner vorgeschlagen, dass die zumindest eine Sendeeinheit zu einer proprietären, unverpackten Übertragung des Signals an die aktive Blendschutzvorrichtung vorgesehen ist. Dadurch kann insbesondere eine vorteilhaft hohe Übertragungsgeschwindigkeit sowie eine vorteilhaft hohe Übertragungssicherheit erreicht werden. Durch die unverpackte Übertragung kann insbesondere auf zusätzliche Informationen, wie z.B. CRC und Syncword, welche bei einer paketorientierten Übertragung benötigt werden, verzichtet werden. Das Übertragungspaket kann daher wesentlich kürzer ausgeführt werden. Hierdurch kann eine vorteilhaft schnelle Datenübertragung erreicht werden. Unter einer "unverpackten Übertragung" soll in diesem Zusammenhang insbesondere eine Übertragung verstanden werden, bei welcher eine Übertragung unverpackt erfolgt. Eine Übertragung erfolgt daher nicht paketorientiert, sodass keine CRCs und/oder Syncwors verwendet werden.

Es wird weiter vorgeschlagen, dass die zumindest eine Kommunikationseinheit zu einer unidirektionalen Übertragung zumindest einer Information des zumindest einen Schweißparameters an die aktive Blendschutzvorrichtung vorgesehen ist. Vorzugsweise erfolgt die Übertragung der zumindest einen Information des zumindest einen Schweißparameters an die aktive Blendschutzvorrichtung in einem Streaming-Verfahren. Dadurch kann insbesondere eine vorteilhaft sichere und zuverlässige Übertragung erreicht werden. Es können Verzögerungen bei der Signalübertragung vermieden werden. Vorzugsweise kann dauerhaft ein Signal an die aktive Blendschutzvorrichtung übertragen werden.

Ferner wird vorgeschlagen, dass eine Bitfolge der Kommunikationseinheit zumindest 4 bit, vorzugsweise zumindest 6 bit, umfasst. Vorzugsweise umfasst eine Bitfolge der Kommunikationseinheit weniger als 32 bit und besonders bevorzugt weniger als 16 bit. Bevorzugt umfasst die Bitfolge der Kommunikationseinheit genau 8 bit. Grundsätzlich wäre jedoch auch eine andere, einem Fachmann als sinnvoll erscheinende Anzahl an Bits denkbar. Dadurch kann eine vorteilhaft sichere Übertragung gewährleistet werden. Hierdurch kann auch in verrauschter Umgebung ein gültiges Signal eindeutig rekonstruiert werden. Die Robustheit der Datenübertragung nimmt mit zunehmender Länge der Bitfolge zu. Dies geht jedoch auf Kosten der erreichbaren minimalen Latenzzeiten. Für den jeweiligen Anwendungsfall kann die Bitfolge insbesondere angepasst werden.

Des Weiteren wird vorgeschlagen, dass die zumindest eine Modulationseinheit zu einer Modulation des Signal der zumindest einen Sendeeinheit mit einer Modulationsrate von zumindest 100 kbit/s, vorzugsweise von zumindest 400 kbit/s und besonders bevorzugt von zumindest 800 kbit/s, vorgesehen ist. Hierdurch kann insbesondere eine vorteilhaft schnelle Datenübertragung erreicht werden. Es kann insbesondere eine vorteilhaft hohe Übertragungsgeschwindigkeit sowie eine vorteilhaft hohe Übertragungssicherheit erreicht werden.

Es wird weiter vorgeschlagen, dass die zumindest eine Modulationseinheit zu einer direkten Modulation des Signals der zumindest einen Sendeeinheit zumindest einen HF-Transceiverbaustein aufweist. Vorzugsweise ist der HF-Transceiverbaustein von einem HF-Transceiverbaustein mit direkter Modulation gebildet. Hierdurch kann eine vorteilhafte Modulationseinheit bereitgestellt werden.

Ferner wird vorgeschlagen, dass die Erfassungseinheit zu einer direkten oder indirekten Erfassung von zumindest zwei Schaltzuständen des Schweißgeräts vorgesehen ist. Vorzugsweise ist die Erfassungseinheit zu einer direkten oder indirekten Erfassung von zumindest einem Schweißbetriebszustand und zumindest einem Ruhezustand, insbesondere den Zuständen "schweißen inaktiv" und "schweißen aktiv", des Schweißgeräts vorgesehen. Dadurch kann eine vorteilhafte Erfassung erreicht werden. Vorzugsweise kann dadurch insbesondere zu einer Frühabdunkelung des aktiven Blendschutzfilters zuverlässig und insbesondere unabhängig von dem Schweißgerät ein Zustand des Schweißgeräts erfasst werden.

Zudem wird vorgeschlagen, dass die zumindest eine Kommunikationseinheit dazu vorgesehen ist, die zwei Schaltzustände des Schweißgeräts in Form von zueinander inversen Bitfolgen direkt an die aktive Blendschutzvorrichtung zu übertragen. Vorzugsweise ist jedem Schaltzustand jeweils eine Bitfolge zugeordnet, wobei die Bitfolgen jeweils invers zueinander sind, also jeder Bit der Bitfolge den gegenteiligen Wert aufweist. Hierdurch kann jede Bitfolge auch bei einer verrauschten Umgebung und nicht auslesbarer Bits eindeutig zugeordnet werden. Diese Darstellung ermöglicht es, eventuelle Einflüsse von Störsignalen mittels Signalverarbeitung gering zu halten. Die Übertragungszeit eines Signalzustands wird dadurch zwar entsprechend vergrößert, liegt jedoch weiterhin unterhalb einer paketorientierten Übertragung.

Ferner geht die Erfindung aus von einem System mit der aktiven Blendschutzvorrichtung und mit der Erfassungsvorrichtung.

Die erfindungsgemäße Erfassungsvorrichtung und das entsprechende System sollen hierbei nicht auf die oben beschriebene Anwendung und Ausführungsform beschränkt sein. Insbesondere können die erfindungsgemäße Erfassungsvorrichtung, das System sowie das Verfahren zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein System mit einer aktiven Blendschutzvorrichtung und mit einer erfindungsgemäßen Erfassungsvorrichtung und ein Schweißgerät in einer schematischen Darstellung,
- Fig. 2: die erfindungsgemäße Erfassungsvorrichtung mit einer Erfassungseinheit und mit einer Kommunikationseinheit in einer schematischen Vorderansicht,
- Fig. 3: die erfindungsgemäße Erfassungsvorrichtung mit der Erfassungseinheit und mit der Kommunikationseinheit in einer schematischen Rückansicht,
- Fig. 4: eine veranschaulichte Paketstruktur der Kommunikationseinheit der erfindungsgemäßen Erfassungsvorrichtung in einer schematischen Darstellung,
- Fig. 5: eine veranschaulichte Datenabtastung eines Signals der Kommunikationseinheit der erfindungsgemäßen Erfassungsvorrichtung durch eine Kommunikationseinheit der aktiven Blendschutzvorrichtung in einer schematischen Darstellung und
- Fig. 6: ein schematisches Ablaufdiagramm eines Verfahrens zu einem Betrieb der erfindungsgemäßen Erfassungsvorrichtung.

### Beschreibung des Ausführungsbeispiels

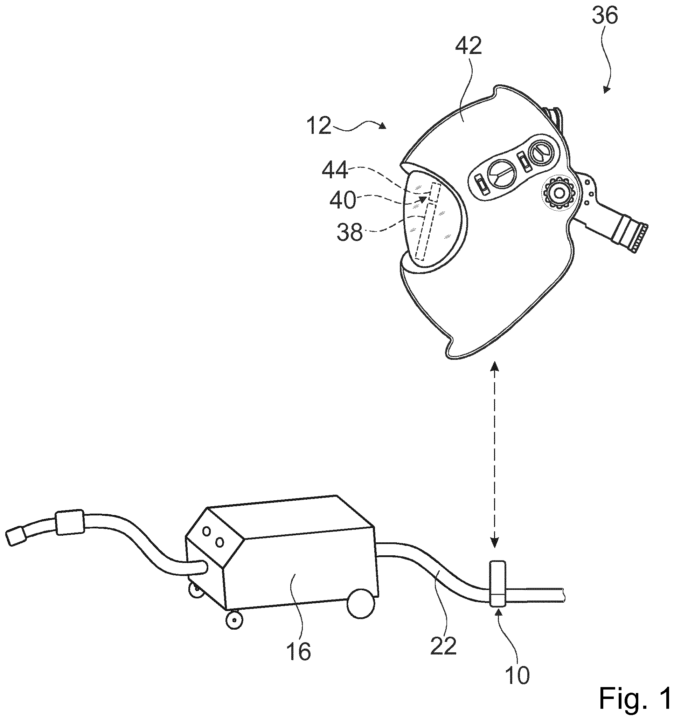

Figur 1 zeigt eine aktiven Blendschutzvorrichtung 12, eine Erfassungsvorrichtung 10 und ein Schweißgerät 16. Das Schweißgerät 16 ist von einem elektrisch betriebenen Schweißgerät gebildet. Das Schweißgerät 16 ist von einem Lichtbogenschweißgerät gebildet. Grundsätzlich wäre jedoch auch eine andere, einem Fachmann als sinnvoll erscheinende Ausbildung des Schweißgeräts 16 denkbar.

Die Blendschutzvorrichtung 12 und die Erfassungsvorrichtung 10 bilden ein System 36 aus. Die Blendschutzvorrichtung 12 ist dazu vorgesehen, während eines Betriebs von einem Bediener auf dem Kopf getragen zu werden. Die Blendschutzvorrichtung 12 ist von einem Schweißhelm gebildet. Grundsätzlich wäre jedoch auch eine andere, einem Fachmann als sinnvoll erscheinende Ausbildung der Blendschutzvorrichtung 12 denkbar. Die Erfassungsvorrichtung 10 ist von der Blendschutzvorrichtung 12 getrennt ausgebildet.

Die Blendschutzvorrichtung 12 weist einen optischen Blendschutzfilter 38 auf. Der Blendschutzfilter 38 ist dazu vorgesehen, abhängig von einem Arbeitszustand und einer davon abhängigen Ansteuerung eine Durchlässigkeit zu verändern. Der optische Blendschutzfilter 38 ist von einem elektrooptischen Filter gebildet. Der optische Blendschutzfilter 38 ist von einem automatic darkening filter, kurz ADF, gebildet. Der optische Blendschutzfilter 38 besteht aus mehreren Schichten. Der optische Blendschutzfilter 38 ist als ein Mehrschichtverbund ausgebildet. Die Flüssigkristallschicht des Blendschutzfilters 38 wird während eines Betriebs von der Recheneinheit 40 abgedunkelt, wenn ein Schweißverfahren erfasst wird. Der Blendschutzfilter 38 weist eine rechteckige Grundform auf.

Ferner weist die Blendschutzvorrichtung 12 eine Schildeinheit 42 auf. Der Blendschutzfilter 38 ist fest in der Schildeinheit 42 aufgenommen. Der Blendschutzfilter 38 ist positionsfest in der Schildeinheit 42 aufgenommen. Die Blendschutzvorrichtung 12 weist ferner eine Vorsatzscheibe auf. Die Vorsatzscheibe ist mit der Schildeinheit 42 über nicht weiter sichtbare Rastelemente verbunden. Die Vorsatzscheibe ist transparent ausgebildet. Die Vorsatzscheibe ist zu einem Schutz des Blendschutzfilters 38 vorgesehen. Die Vorsatzscheibe verdeckt den Blendschutzfilter 38 von außen.

Des Weiteren weist die Blendschutzvorrichtung 12 eine Kommunikationseinheit 44 auf. Die Kommunikationseinheit 44 ist zu einer Kommunikation mit der von der Blendschutzvorrichtung 12 getrennten Erfassungsvorrichtung 10 vorgesehen. Die Kommunikationseinheit 44 ist zu einem Empfang von Signalen der von der Blendschutzvorrichtung 12 getrennten Erfassungsvorrichtung 10 vorgesehen. Die Kommunikationseinheit 44 ist in die Recheneinheit 40 integriert. Die Kommunikationseinheit 44 bildet ein Teil der Recheneinheit 40. Grundsätzlich wäre jedoch auch eine separate Ausbildung der Kommunikationseinheit 44 denkbar. Über die Kommunikationseinheit 44 kann die Blendschutzvorrichtung 12 Signale zu einer Abdunkelung des Blendschutzfilters 38 empfangen. Über die Kommunikationseinheit 44 kann die Blendschutzvorrichtung 12 mittels der Erfassungsvorrichtung 10 erfasste Schweißparameter A des Schweißgeräts 16 empfangen. Die Schweißparameter A des Schweißgeräts 16 werden von der Erfassungsvorrichtung 10 an die Blendschutzvorrichtung 12 übertragen. Die Recheneinheit 40 der Blendschutzvorrichtung 12 ist zu einer Auswertung der Schweißparameter A des Schweißgeräts 16 vorgesehen. Ferner ist die Recheneinheit 40 dazu vorgesehen, abhängig von den Schweißparametern A des Schweißgeräts 16 den Blendschutzfilter 38 abzudunkeln. Hierbei wäre es insbesondere denkbar, dass die Recheneinheit 40 den Blendschutzfilter 38 bei einem Ausbleiben eines Signals von der Erfassungsvorrichtung 10 den Blendschutzfilter 38 automatisch abdunkelt. Hierdurch könnte bei einem Ausbleiben eines Signals von der Erfassungsvorrichtung 10, beispielsweise aufgrund einer zu großen Entfernung zwischen der Blendschutzvorrichtung 12 und der Erfassungsvorrichtung 10, eine Schädigung des Bedieners ausgeschlossen werden. Zudem oder alternativ wäre jedoch auch denkbar, dass die Recheneinheit 40 einen zusätzlichen, insbesondere optischen, Sensor zu einer Erfassung eines Lichtbogens des Schweißgeräts 16 aufweist, mittels welchem der Blendschutzfilter 38 ebenfalls abgedunkelt werden kann.

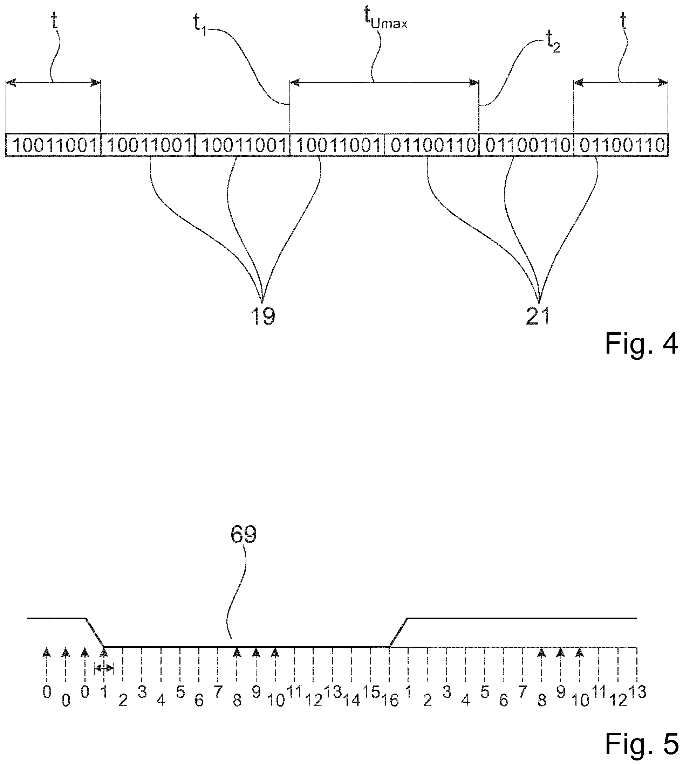

Die Erfassungsvorrichtung 10 ist für die aktive Blendschutzvorrichtung 12 vorgesehen. Die Erfassungsvorrichtung 10 bildet ein Zusatzbauteil für die Blendschutzvorrichtung 12. Die Erfassungsvorrichtung 10 und die Blendschutzvorrichtung 12 sind zu einer gemeinsamen Benutzung vorgesehen. Die Erfassungsvorrichtung 10 weist eine Erfassungseinheit 14 auf. Die Erfassungseinheit 14 ist zu einer direkten oder indirekten Erfassung zumindest eines Schweißparameters A des Schweißgeräts 16 vorgesehen. Die Erfassungseinheit 14 ist zu einer indirekten Erfassung eines Stroms des Schweißgeräts 16 vorgesehen. Die Erfassungseinheit 14 ist zu einer Erfassung eines zu dem Schweißgerät 16 fließenden Stroms vorgesehen. Die Erfassungseinheit 14 ist zu einer indirekten Erfassung von zwei Schaltzuständen des Schweißgeräts 16 vorgesehen. Das Schweißgerät 16 weist zwei Schaltzustände auf. Das Schweißgerät 16 weist einen Schweißbetriebszustand, welcher insbesondere für "schweißen aktiv" steht, und einen Ruhezustand, welcher insbesondere für "schweißen inaktiv" steht, auf. Die Erfassungseinheit 14 ist zu einer indirekten Erfassung von dem Schweißbetriebszustand und dem Ruhezustand des Schweißgeräts 16 vorgesehen. Über den Schweißparameter A schließt die Erfassungseinheit 14 auf einen Schaltzustand, in welchem sich das Schweißgerät 16 befindet. Die Erfassungseinheit 14 weist einen Stromsensor 20 auf. Der Stromsensor 20 ist zu einer Erfassung des Schweißparameters A des Schweißgeräts 16 vorgesehen. Der Stromsensor 20 ist zu einer Erfassung eines von einem Strom gebildeten Schweißparameters A des Schweißgeräts 16 vorgesehen. Der Stromsensor 20 ist zu einer Erfassung des Schweißparameters A des Schweißgeräts 16 dazu vorgesehen, variabel an ein Stromkabel 22 des Schweißgeräts 16 befestigt zu werden. Das Stromkabel 22 des Schweißgeräts 16 ist von einem Brenner- und/oder Massekabel gebildet. Der Stromsensor 20 ist zu einer Erfassung des zumindest einen Schweißparameters A des Schweißgeräts 16 dazu vorgesehen, elektrisch kontaktlos, variabel an das Stromkabel 22 des Schweißgeräts 16 befestigt zu werden. Der Stromsensor 20 ist in einem Betrieb variabel an ein Stromkabel 22 des Schweißgeräts 16 befestigt. Die gesamte Erfassungsvorrichtung 10 ist in einem Betrieb variabel an ein Stromkabel 22 des Schweißgeräts 16 befestigt. Die Erfassungsvorrichtung 10 umgreift dazu das Stromkabel 22 des Schweißgeräts 16. Die Erfassungsvorrichtung 10 wird dazu um das Stromkabel 22 des Schweißgeräts 16 herum befestigt. Die Erfassungsvorrichtung 10 bildet eine Schelle aus. Die Erfassungsvorrichtung 10 kann an einer beliebigen Position an dem Stromkabel 22 des Schweißgeräts 16 sowie an einem beliebigen Schweißgerät 16 befestigt werden. Die Erfassungsvorrichtung 10 ist unabhängig von einem Schweißgerät 16 funktionsfähig und kann insbesondere an Schweißgeräte 16 verschiedener Hersteller befestigt werden (Figuren 1, 5).

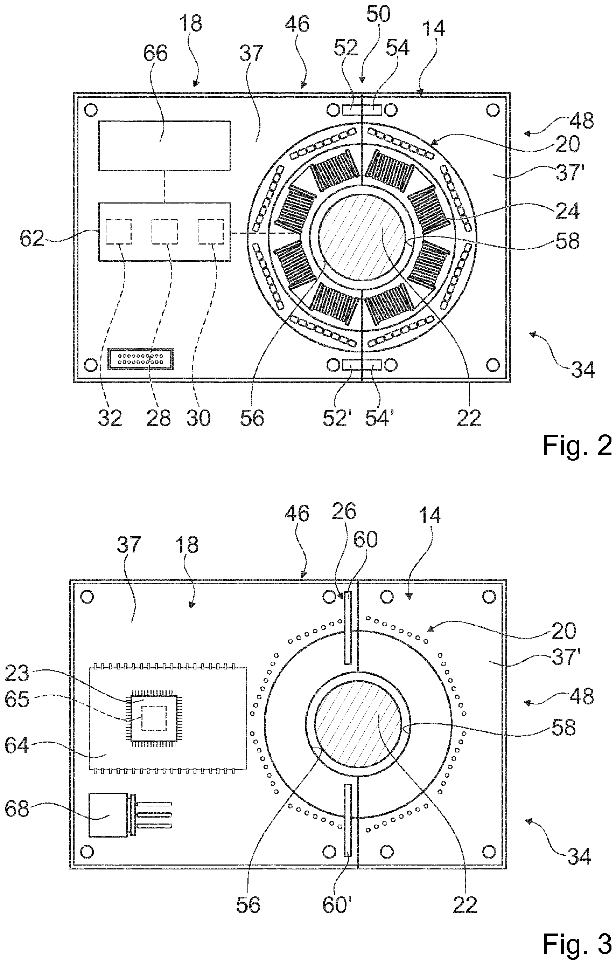

Die Erfassungsvorrichtung 10 weist ein mehrteiliges Gehäuse 34 auf. Die Erfassungsvorrichtung 10 weist ein zweiteiliges Gehäuse 34 auf. Das Gehäuse 34 nimmt die Erfassungseinheit 14 der Erfassungsvorrichtung 10 auf. Das Gehäuse 34 ist dazu vorgesehen, werkzeuglos um das Stromkabel 22 des Schweißgeräts 16 herum montiert zu werden. Das Gehäuse 34 weist eine erste Gehäusehälfte 46 und eine zweite Gehäusehälfte 48 auf. Die Gehäusehälften 46, 48 weisen jeweils auf einander zugewandten Seiten eine Aussparung 56, 58 für das Stromkabel 22 auf. Die Aussparungen 56, 58 der Gehäusehälften 46, 48 sind jeweils halbkreisförmig ausgebildet. Die Aussparungen 56, 58 bilden gemeinsam eine kreisförmige Aussparung. Die Gehäusehälften 46, 48 sind über eine Verbindungseinheit 50 miteinander verbindbar. Die Verbindungseinheit 50 umfasst zwei erste Verbindungselemente 52, 52', welche fest mit der ersten Gehäusehälfte 46 verbunden sind, und zwei zweite Verbindungselemente 54, 54', welche fest mit der zweiten Gehäusehälfte 48 verbunden sind. Die ersten und zweiten Verbindungselemente 52, 52', 54, 54' werden zu einer Verbindung der Gehäusehälften 46, 48 miteinander verschraubt. Grundsätzlich wäre jedoch auch eine andere, einem Fachmann als sinnvoll erscheinende Verbindungstechnik denkbar, wie beispielsweise magnetisch. Zu einer Montage können die Gehäusehälften 46, 48 der Erfassungsvorrichtung 10 daher getrennt und um das Stromkabel 22 herum wieder geschlossen werden (Figuren 2, 3).

Ferner weist die Erfassungsvorrichtung 10 eine mehrteilige Platine 35 auf. Die Platine 35 ist zweiteilig ausgebildet. Die Platine 35 weist zwei getrennte Platinenelemente 37, 37' auf. Die Platinenelemente 37, 37' sind korrespondierend zu den Gehäusehälften 46, 48 ausgebildet. Das erste Platinenelement 37 ist in der ersten Gehäusehälfte 46 angeordnet. Das zweite Platinenelement 37' ist in der zweiten Gehäusehälfte 48 angeordnet. Die Platinenelemente 37, 37' sind dazu vorgesehen, um das Stromkabel 22 des Schweißgeräts 16 herum montiert zu werden. Die Platinenelemente 37, 37' weisen dazu zu den Aussparungen 56, 58 der Gehäusehälften 46, 48 korrespondierende Aussparungen auf.

Ferner weist der Stromsensor 20 der Erfassungseinheit 14 ein erstes Sensorelement 24 auf. Das erste Sensorelement 24 ist zu einer Erfassung einer Stromänderung in dem Stromkabel 22 des Schweißgeräts 16 vorgesehen. Das erste Sensorelement 24 umgreift in einem Betrieb das Stromkabel 22 des Schweißgeräts 16. Das erste Sensorelement 24 des Stromsensor 20 ist um die Aussparungen 56, 58 der Gehäusehälften 46, 48 angeordnet. Das erste Sensorelement 24 ist von einer Rogowskispule gebildet. Das erste Sensorelement 24 ist von einer Rogowskispule gebildet, welche sich in einem Betrieb ringförmig um das Stromkabel 22 erstreckt. Vorzugsweise ist die Rogowskispule aus mehreren Teilspulen zusammengesetzt. Das erste Sensorelement 24 ist teilweise auf das erste Platinenelement 37 und teilweise auf das zweite Platinenelement 37' aufgebracht. Das erste Sensorelement 24 besteht aus mehreren Luftspulen, mit welchen die Platinenelemente 37, 37' bestückt sind. Das erste Sensorelement 24 besteht aus mehreren SMD bestückbaren Luftspulen (Figuren 2, 3).

Des Weiteren weist der Stromsensor 20 der Erfassungseinheit 14 ein zweites Sensorelement 26 auf. Das zweite Sensorelement 26 ist zu einer Erfassung eines absoluten Stromwerts in dem Stromkabel 22 des Schweißgeräts 16 vorgesehen. Mit dem zweiten Sensorelement 26 ist daher insbesondere auch ein anhaltender Stromwert messbar. Das zweite Sensorelement 26 weist zwei Messelemente 60, 60' auf, welche auf gegenüberliegenden Seiten der Aussparungen 56, 58 der Gehäusehälften 46, 48 angeordnet sind. Die Messelemente 60, 60' sind in einem Betrieb auf gegenüberliegenden Seiten des Stromkabels 22 angeordnet. Das zweite Sensorelement 26 ist von einem Hallsensor gebildet. Die Messelemente 60, 60' des zweiten Sensorelements 26 sind jeweils von Hallsensorboards gebildet (Figur 3).

Die Erfassungsvorrichtung 10 weist ferner eine Kommunikationseinheit 18 auf. Die Kommunikationseinheit 18 ist zu einer Übertragung zumindest einer Information des Schweißparameters A an die aktive Blendschutzvorrichtung 12 vorgesehen. Die Kommunikationseinheit 18 der Erfassungsvorrichtung 10 ist zu einer Kommunikation mit der Kommunikationseinheit 44 der Blendschutzvorrichtung 12 vorgesehen. Die Kommunikationseinheit 18 der Erfassungsvorrichtung 10 ist zu einer Übertragung einer aus dem Schweißparameter A resultierenden Information an die Kommunikationseinheit 44 der Blendschutzvorrichtung 12 vorgesehen. Die Kommunikationseinheit 18 der Erfassungsvorrichtung 10 ist zu einer Übertragung des aktuellen Schaltzustands des Schweißgeräts 16 an die Kommunikationseinheit 44 der Blendschutzvorrichtung 12 vorgesehen. Die Kommunikationseinheit 18 ist dazu vorgesehen, die Information des Schweißparameters A, insbesondere den Schaltzustand, in Form einer Bitfolge 19, 21 definierter Länge an die aktive Blendschutzvorrichtung 12 zu übertragen. Die Kommunikationseinheit 18 ist dazu vorgesehen, die Information des Schweißparameters A, insbesondere den Schaltzustand, in Form einer Bitfolge 19, 21 definierter Länge kabellos an die aktive Blendschutzvorrichtung 12 zu übertragen. Die Kommunikationseinheit 18 ist zu einer unidirektionalen Übertragung der Information des zumindest einen Schweißparameters A, insbesondere den Schaltzustand, an die aktive Blendschutzvorrichtung 12 vorgesehen. Jedem der zwei Schaltzustände des Schweißgeräts 16 ist dabei eine feste Bitfolge 19, 21 zugeordnet. Die Bitfolgen 19, 21 weisen dieselbe Anzahl an Bits auf. Die Bitfolgen 19, 21 weisen jeweils mehr als ein Bit auf. Die Kommunikationseinheit 18 ist dazu vorgesehen, die zwei Schaltzustände des Schweißgeräts 16 in Form von zueinander inversen Bitfolgen 19, 21 direkt an die aktive Blendschutzvorrichtung 12 zu übertragen. Die Bitfolgen 19, 21 sind jeweils invers, sodass jeder Bit der Bitfolge 19, 21 den gegenteiligen Wert des Bits der anderen Bitfolge 19, 21 an derselben Position aufweist. Die Bitfolgen 19, 21 der Kommunikationseinheit 18 umfassen jeweils zumindest 4 bit. Die Bitfolgen 19, 21 der Kommunikationseinheit 18 umfassen jeweils genau 8 bit. Grundsätzlich wäre auch eine andere, einem Fachmann als sinnvoll erscheinende Bitanzahl denkbar. Die Bitanzahl stellt insbesondere ein vorteilhaftes Verhältnis aus Robustheit und Übertragungsgeschwindigkeit dar. Die Robustheit der Datenübertragung nimmt mit zunehmender Länge der Bitfolge 19, 21 zu. Dies geht jedoch auf Kosten der erreichbaren minimalen Latenzzeiten. Für den jeweiligen Anwendungsfall kann die Bitfolge daher angepasst werden.

Die Kommunikationseinheit 18 weist eine Signalverarbeitungseinheit 62 auf. Die Signalverarbeitungseinheit 62 ist von einer analogen Signalverarbeitung gebildet. Die Signalverarbeitungseinheit 62 ist zu einer Verbreitung der Daten der Erfassungseinheit 14 vorgesehen. Die Signalverarbeitungseinheit 62 ist zu einer Verbreitung der Daten der Erfassungseinheit 14 vor einem Versenden vorgesehen. Die Signalverarbeitungseinheit 62 ist direkt mit der Erfassungseinheit 14 gekoppelt. Die Signalverarbeitungseinheit 62 umfasst einen Differentialverstärker 28, einen Gleichrichter 30 und einen Komparator 32. Ferner weist die Kommunikationseinheit 18 eine Sendeeinheit 64 auf. Die Sendeeinheit 64 ist von einem Funkmodul gebildet. Die Sendeeinheit 64 ist von einem RF-Modul gebildet. Die Sendeeinheit 64 ist von einem System-on-a-Chip-RF-Modul, kurz RF-SoC-Modul, gebildet. Die Sendeeinheit 64 ist zu einer direkten Übertragung eines Signals an die aktive Blendschutzvorrichtung 12 vorgesehen. Die Sendeeinheit 64 ist zu einer direkten Übertragung eines Funksignals an die aktive Blendschutzvorrichtung 12 vorgesehen. Die Sendeeinheit 64 ist zu einer proprietären, unverpackten Übertragung des Signals an die aktive Blendschutzvorrichtung 12 vorgesehen.

Des Weiteren weist die Kommunikationseinheit 18 eine Modulationseinheit 23 auf. Die Modulationseinheit 23 ist dazu vorgesehen, den zumindest einen Schweißparameter A in Form einer Bitfolge 19, 21 definierter Länge direkt auf das Signal der Sendeeinheit 64 zu modulieren. Die Modulationseinheit 23 ist zu einer Modulation des Signal der einen Sendeeinheit 64 mit einer Modulationsrate von zumindest 100 kbit/s vorgesehen. Die Modulationseinheit 23 ist zu einer Modulation des Signal der einen Sendeeinheit 64 mit einer Modulationsrate von annähernd 1 Mbit/s vorgesehen. Die Modulationseinheit 23 weist zu einer direkten Modulation des Signals der Sendeeinheit 64 einen HF-Transceiverbaustein 65 auf. Die Modulationseinheit 23 weist einen HF-Transceiverbaustein 65 mit direkter Modulation bzw. Demodulation auf. Es wird jedoch keine synchrone Modulation bzw. Demodulation vorausgesetzt. Für eine einfache Implementierung des Streamingverfahrens der Kommunikationseinheit 18 kann als HF-Transceiverbaustein 65 ein standardmässiger UART, also ein Universal Asynchronous Receiver Transmitter, eines Mikrokontrollers der Signalverarbeitungseinheit 62 der Kommunikationseinheit 18 verwendet werden. Das Sendesignal wird durch das wiederholte Übertragen der festgelegten Bitfolge 19, 21 generiert. Beim Umschalten in den anderen Schaltzustand wird die invertierte Bitfolge 19, 21 gesendet. Das Ausgangssignal der UART dient der direkten Modulation der Sendeeinheit 64. Das Zeitverhalten wird durch die standardisierte Datenstruktur der UART eingeschränkt. Entsprechend dem Datenprotokoll wird mindestens ein Start-Bit 69 und ein Stop-Bit vorausgesetzt. Die Übertragungsdauer erhöht sich entsprechend.

Figur 4 zeigt eine veranschaulichte Paketstruktur der Kommunikationseinheit 18 der Erfassungsvorrichtung 10. Die Informationen werden dabei in Form der Bitfolgen 19, 21 übersandt, wobei jede Bitfolge 19, 21 für eine Information steht. Durch die definierte Anzahl an Bits der Bitfolgen 19, 21 sowie durch die definierte Modulationsrate weisen die Bitfolgen 19, 21 jeweils eine definierte Übertragungszeit t auf. Die Übertragungszeit t beträgt in diesem Ausführungsbeispiel 8 µs. Die maximale Umschaltzeit t_{Umax} von einem zum anderen Signalzustand liegt daher bei der doppelten Übertragungszeit t einer Bitfolge 19, 21. Wird beispielhaft zu einem Zeitpunkt t₁, zu Beginn einer Übertragung einer Bitfolge 19, das Schweißgerät 16 von dem Schweißbetriebszustand, welcher von der Kommunikationseinheit 18 durch die erste Bitfolge 19 dargestellt wird, in dem Ruhezustand, welcher von der Kommunikationseinheit 18 durch die zweite Bitfolge 21 dargestellt wird, umgeschalten, kann erst nach der fertigen Übertragung der ersten Bitfolge 19 die zweite Bitfolge 21 übertragen werden. Die zweite Bitfolge 21 muss vollständig übertragen werden, bevor die Information, dass sich das Schweißgerät 16 in dem Ruhezustand befindet, durch die Kommunikationseinheit 44 der Blendschutzvorrichtung 12 zu einem Zeitpunkt t₂ erfasst wird. Aus dem maximal möglichen Abstand von t₁ zu t₂ ergibt sich die maximale Umschaltzeit t_{Umax}.

Des Weiteren weist die Kommunikationseinheit 18 ein Netzteil 66 auf. Das Netzteil 66 dient zu einer Energieversorgung. Das Netzteil 66 ist über einen Batterieanschluss 68 mit einem nicht weiter sichtbaren Energiespeicher verbunden (Figuren 2, 3).

Die Kommunikationseinheit 44 der Blendschutzvorrichtung 12 ist zu einem Empfang des Signals der Sendeeinheit 64 sowie der darauf modulierten Bitfolge 19, 21 der Modulationseinheit 23 vorgesehen. An der Kommunikationseinheit 44 der Blendschutzvorrichtung 12 wird das demodulierte Signal der Erfassungsvorrichtung 10 einem Empfangsbuffer einer UART der Recheneinheit 40 der Blendschutzvorrichtung 12 zugeführt. Es wird keine Bit- oder Datensynchronisation des Demodulators benötigt. Ein Oversampling sowie ein Dekodieren von Empfangsdaten auf Bit-Ebene erfolgt durch die integrierte UART eines Mikrokontrollers der Recheneinheit 40 der Blendschutzvorrichtung 12. Es muss auf Seite der Kommunikationseinheit 18 der Erfassungsvorrichtung 10 und auf Seite der Kommunikationseinheit 44 der Blendschutzvorrichtung 12 für die UART die gleiche Bitrate verwendet werden. Die Korrelation der Empfangsdaten hinsichtlich der zu erwartenden Bitfolge 19, 21 erfolgt übergeordnet in der Recheneinheit 40. Damit bestehen keine Anforderungen an die Kommunikationseinheit 44 der Blendschutzvorrichtung 12 bzw. an die UART hinsichtlich einer Bit-Clock-Rückgewinnung. Hierdurch können die Anforderungen an einen Demodulator vorteilhaft gering gehalten werden. Figur 5 zeigt eine veranschaulichte Datenabtastung, insbesondere eine Startbit-Erkennung, eines Signals der Kommunikationseinheit 18 der Erfassungsvorrichtung 10 durch eine Kommunikationseinheit 44 der aktiven Blendschutzvorrichtung 12. Die Kommunikationseinheit 44 der aktiven Blendschutzvorrichtung 12 tastet dazu insbesondere jeden Bit der Bitfolge 19, 21 16-mal ab. Ist beispielsweise eine Abtastung "1" die erste Nullabtastung, verwendet die Kommunikationseinheit 44 die Abtastungen "8", "9" und "10", um zu entscheiden, ob ein gültiges Start-Bit 69 empfangen wurde. Anschließend wird ein Wert der weiteren Bits der Bitfolge 19, 21 ebenfalls über die Abtastungen "8", "9" und "10" erfasst.

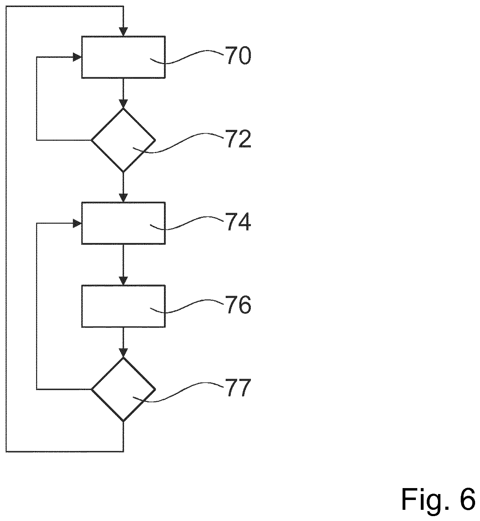

Figur 6 zeigt ein schematisches Ablaufdiagramm eines Verfahrens zu einem Betrieb der erfindungsgemäßen Erfassungsvorrichtung 10. Bei dem Verfahren wird in einem ersten Verfahrensschritt 70 mittels der Erfassungseinheit 14 eine in dem Stromkabel 22 des Schweißgeräts 16 vorliegende Stromänderung gemessen. In dem ersten Verfahrensschritt 70 wird mit dem ersten Sensorelement 24 einschließlich der Signalverarbeitungseinheit 62 ständig eine Stromänderung erfasst. Der erste Verfahrensschritt 70 bildet einen Ruhezustand aus. Das Schweißgerät 16 befindet sich daher in dem Ruhezustand. In dem ersten Verfahrensschritt 70 wird über die Kommunikationseinheit 18 kontinuierlich ein Statussignal "schweißen inaktiv" an die Blendschutzvorrichtung 12 übertragen. Das Statussignal "schweißen inaktiv" wird durch Senden der zweiten Bitfolge 21 an die Blendschutzvorrichtung 12 übertragen. Die Modulationseinheit 23 moduliert dazu die zweite Bitfolge 21 auf das Signal der Sendeeinheit 64. Durch die ständige Übertragung eines Status mittels der Bitfolge 21 kann eine Verbindung zwischen der Erfassungsvorrichtung 10 und der Blendschutzvorrichtung 12 überwacht werden. Anschließend wird in einer ersten Verzweigung 72 überwacht, ob eine Stromänderung in dem Stromkabel 22 einen festgelegten Grenzwert überschreitet. Bleibt eine Änderung des Stroms unterhalb des Grenzwerts, wird der erste Verfahrensschritt 70 wiederholt. Übersteigt die erfasste Stromänderung den Grenzwert, wird dies in einem zweiten Verfahrensschritt 74 sofort an die Kommunikationseinheit 18 übertragen, welche sofort ein Statussignal "schweißen aktiv" an die Blendschutzvorrichtung 12 überträgt. Das Statussignal "schweißen aktiv" wird durch Senden der ersten Bitfolge 19 an die Blendschutzvorrichtung 12 übertragen. Die Modulationseinheit 23 moduliert dazu die erste Bitfolge 19 auf das Signal der Sendeeinheit 64. Anschließend wird die Blendschutzvorrichtung 12 in einem dritten Verfahrensschritt 76 sofort abgedunkelt. Darauffolgend wird in einer zweiten Verzweigung 77 der absolute Stromwert in dem Stromkabel 22 erfasst. Die Erfassung des absoluten Stromwerts erfolgt mittels des zweiten Sensorelements 26. Wenn der absolute Stromwert einen Grenzwert überschreitet, wird der zweite Verfahrensschritt 74 wiederholt und es wird kontinuierlich durch Senden der ersten Bitfolge 19 das Statussignal "schweißen aktiv" an die Blendschutzvorrichtung 12 gesendet. Die Blendschutzvorrichtung 12 bleibt daher abgedunkelt. Wird der Grenzwert nicht überschritten, wird der erste Verfahrensschritt 70 wiederholt. Die Erfassungseinheit 14 geht daher wieder in den Ruhezustand und die Kommunikationseinheit 18 sendet durch Senden der zweiten Bitfolge 21 das Statussignal "schweißen inaktiv". Die Blendschutzvorrichtung 12 wird wieder geöffnet. Nach der Übertragung öffnet die Blendschutzvorrichtung 12 daher wieder. Das Öffnen der Blendschutzvorrichtung 12 ist dabei insbesondere nicht so zeitkritisch und kann nach einigen Millisekunden erfolgen. Damit ist das ungünstigere Zeitverhalten des zweiten Sensorelements 26 für die Erfassung des Schweißendes ausreichend. Da die Funkübertragung immer kontinuierlich erfolgt, ist neben der zeitnahen Übertragung eines neuen Zustands auch eine Überwachung der Funkverbindung möglich.

## Patentansprüche

1. Erfassungsvorrichtung für eine aktive Blendschutzvorrichtung (12), mit einer Erfassungseinheit (14), welche zu einer direkten oder indirekten Erfassung zumindest eines Schweißparameters (A) eines Schweißgeräts (16) vorgesehen ist, und mit zumindest einer Kommunikationseinheit (18), welche zu einer Übertragung zumindest einer Information des zumindest einen Schweißparameters (A) an die aktive Blendschutzvorrichtung (12) vorgesehen ist, wobei die Kommunikationseinheit (18) dazu vorgesehen ist, die zumindest eine Information des zumindest einen Schweißparameters (A) in Form einer Bitfolge (19, 21) definierter Länge an die aktive Blendschutzvorrichtung (12) zu übertragen, wobei die zumindest eine Kommunikationseinheit (18) zu einer unidirektionalen Übertragung zumindest einer Information des zumindest einen Schweißparameters (A) an die aktive Blendschutzvorrichtung (12) vorgesehen ist, wobei die Übertragung der zumindest einen Information des zumindest einen Schweißparameters (A) an die aktive Blendschutzvorrichtung in einem Streaming-Verfahren erfolgt, wobei die Erfassungseinheit (14) zu einer direkten oder indirekten Erfassung von zumindest zwei Schaltzuständen des Schweißgeräts (16) vorgesehen ist, wobei die zumindest eine Kommunikationseinheit (18) dazu vorgesehen ist, die zwei Schaltzustände des Schweißgeräts (16) in Form von zueinander inversen Bitfolgen (19, 21) direkt an die aktive Blendschutzvorrichtung (12) zu übertragen.

2. Erfassungsvorrichtung nach Anspruch 1,
**dadurch gekennzeichnet, dass**
die Kommunikationseinheit (18) dazu vorgesehen ist, die zumindest eine Information des zumindest einen Schweißparameters (A) in Form einer Bitfolge (19, 21) definierter Länge kabellos an die aktive Blendschutzvorrichtung (12) zu übertragen.

3. Erfassungsvorrichtung nach Anspruch 1 oder 2,
**dadurch gekennzeichnet, dass**
die Kommunikationseinheit (18) zumindest eine Sendeeinheit (64), welche zu einer direkten Übertragung eines Signals an die aktive Blendschutzvorrichtung (12) vorgesehen ist, und zumindest eine Modulationseinheit (23), welche dazu vorgesehen ist, den zumindest einen Schweißparameter (A) in Form einer Bitfolge (19, 21) definierter Länge direkt auf das Signal zu modulieren, aufweist.

4. Erfassungsvorrichtung nach Anspruch 3,
**dadurch gekennzeichnet, dass**
die zumindest eine Sendeeinheit (64) zu einer proprietären, unverpackten Übertragung des Signals an die aktive Blendschutzvorrichtung (12) vorgesehen ist.

5. Erfassungsvorrichtung nach einem der vorhergehenden Ansprüche,
**dadurch gekennzeichnet, dass**
eine Bitfolge (19, 21) der Kommunikationseinheit (18) zumindest 4 bit umfasst.

6. Erfassungsvorrichtung zumindest nach Anspruch 3,
**dadurch gekennzeichnet, dass**
die zumindest eine Modulationseinheit (23) zu einer Modulation des Signal der zumindest einen Sendeeinheit (64) mit einer Modulationsrate von zumindest 100 kbit/s vorgesehen ist.

7. Erfassungsvorrichtung zumindest nach Anspruch 3,
**dadurch gekennzeichnet, dass**
die zumindest eine Modulationseinheit (23) zu einer direkten Modulation des Signals der zumindest einen Sendeeinheit (64) zumindest einen HF-Transceiverbaustein (65) aufweist.

8. System mit einer aktiven Blendschutzvorrichtung (12) und mit einer Erfassungsvorrichtung (10) nach einem der vorhergehenden Ansprüche.

## Claims

1. Detection device for an active glare protection device (12), with a detection unit (14) that is configured for a direct or indirect detection of at least one welding parameter (A) of a welding apparatus (16), and with at least one communication unit (18) that is configured for a transmission of at least one information of the at least one welding parameter (A) to the active glare protection device (12),
wherein the communication unit (18) is configured to transmit the at least one information of the at least one welding parameter (A) to the active glare protection device (12) as a bit sequence (19, 21) having a defined length,
wherein the at least one communication unit (18) is configured for an unidirectional transmission of at least one information of the at least one welding parameter (A) to the active glare protection device (12),
wherein the transmission of the at least one information of the at least one welding parameter (A) to the active glare protection device (12) is effected in a streaming procedure,
wherein the detection unit (14) is configured for a direct or indirect detection of at least two switch states of the welding apparatus (16),
wherein the at least one communication unit (18) is configured to transmit the two switch states of the welding apparatus (16) directly to the active glare protection device (12) as bit sequences (19, 21) which are inverse to one another.

2. Detection device according to claim 1,
**characterised in that** the communication unit (18) is configured for a wireless transmission of the at least one information of the at least one welding parameter (A) to the active glare protection device (12) as a bit sequence (19, 21) having a defined length.

3. Detection device according to claim 1 or 2,
**characterised in that** the communication unit (18) comprises at least one transmission unit (64), which is configured for a direct transmission of a signal to the active glare protection device (12), and comprises at least one modulation unit (23), which is configured for a modulation of the at least one welding parameter (A) directly onto the signal as a bit sequence (19, 21) having a defined length.

4. Detection device according to claim 3,
**characterised in that** the at least one transmission unit (64) is configured for a proprietary, non-packaged transmission of the signal to the active glare protection device (12).

5. Detection device according to one of the preceding claims,
**characterised in that** a bit sequence (19, 21) of the communication unit (18) comprises at least 4 bits.

6. Detection device at least according to claim 3,
**characterised in that** the at least one modulation unit (23) is configured for a modulation of the signal of the at least one transmission unit (64) with a modulation rate of at least 100 kbit/s.

7. Detection device at least according to claim 3,
**characterised in that** for a direct modulation of the signal of the at least one transmission unit (64), the at least one modulation unit (23) comprises at least one HF transceiver component (65).

8. System with an active glare protection device (12) and with a detection device (10) according to one of the preceding claims.

## Revendications

1. Dispositif de détection pour un dispositif anti-éblouissement actif (12), avec une unité détectrice (14) configurée pour une détection directe ou indirecte d'au moins un paramètre de soudage (A) d'un appareil à soudage (16), et avec au moins une unité de communication (18) configurée pour transmettre au moins une information de l'au moins un paramètre de soudage (A) au dispositif anti-éblouissement actif (12),
où l'unité de communication (18) est configurée pour transmettre l'au moins une information de l'au moins un paramètre de soudage (A) au dispositif anti-éblouissement actif (12) sous forme d'une séquence de bits (19, 21) ayant une longueur définie,
l'au moins une unité de communication (18) étant configurée pour une transmission uni-directionnelle d'au moins une information de l'au moins un paramètre de soudage (A) au dispositif anti-éblouissement actif (12),
la transmission de l'au moins une information de l'au moins un paramètre de soudage (A) au dispositif anti-éblouissement actif (12) s'effectuant par un procédé de streaming,
l'unité détectrice (14) étant configurée pour une détection directe ou indirecte d'au moins deux états de commutation de l'appareil à soudage (16),
l'au moins une unité de communication (18) étant configurée pour transmettre les deux états de commutation de l'appareil à soudage (16) directement au dispositif anti-éblouissement actif (12) sous forme des séquences de bits qui sont inverses l'une à l'autre.

2. Dispositif de détection selon la revendication 1,
**caractérisé en ce que** l'unité de communication (18) est configurée pour une transmission sans fil de l'au moins une information de l'au moins un paramètre de soudage (A) au dispositif anti-éblouissement actif (12) sous forme d'une séquence de bits (19, 21) ayant une longueur définie.

3. Dispositif de détection selon la revendication 1 ou 2,
**caractérisé en ce que** l'unité de communication (18) comporte au moins une unité de transmission (64), qui est configurée pour une transmission directe d'un signal au dispositif anti-éblouissement actif (12), et comporte au moins une unité modulatrice (23), qui est configurée pour moduler l'au moins un paramètre de soudage (A) directement sur le signal sous forme d'une séquence de bits (19, 21) ayant une longueur définie.

4. Dispositif de détection selon la revendication 3,
**caractérisé en ce que** l'au moins une unité de transmission (64) est configurée pour une transmission du signal au dispositif anti-éblouissement actif (12), ladite transmission étant propriétaire et non-conditionnée.

5. Dispositif de détection selon l'une quelconque des revendications précédentes, **caractérisé en ce qu'**une séquence de bits (19, 21) de l'unité de communication (18) comprend au moins 4 bits.

6. Dispositif de détection au moins selon la revendication 3,
**caractérisé en ce que** l'au moins une unité modulatrice (23) est configurée pour une modulation du signal de l'au moins une unité de transmission (64) avec une vitesse de modulation d'au moins 100 kbit/s.

7. Dispositif de détection au moins selon la revendication 3,
**caractérisé en ce que** pour une modulation directe du signal de l'au moins une unité de transmission (64) l'au moins une unité modulatrice (23) comporte au moins un composant émetteur-récepteur HF (65).

8. Système avec un dispositif anti-éblouissement actif (12) et avec un dispositif de détection (10) selon l'une quelconque des revendications précédentes.
